# EUROPÄISCHE PATENTANMELDUNG

(11) **EP 0 587 197 A1**
(43) Veröffentlichungstag der Anmeldung: **16.03.1994**
(21) Anmeldenummer: 93117016.1
(22) Anmeldetag: 11.10.1991
(51) Int. Cl.: A61F 2/04

(54) **Vorrichtung zur Anordnung in einer Körperröhre**

(30) Priorität: 13.10.1990 DE 9014230 U
(62) Teilanmeldung aus: 91117330.0
(71) Anmelder: ANGIOMED AG, D-76227 Karlsruhe (DE)
(72) Erfinder: Lindenberg, Josef, D-76227 Karlsruhe (DE); Schnepp-Pesch, Wolfram, D-76185 Karlsruhe (DE)
(74) Vertreter: Dipl.-Ing. Heiner Lichti, Dipl.-Phys. Dr.rer.nat. Jost Lempert, Dipl.-Ing. Hartmut Lasch

(57) **Zusammenfassung**

Es wird eine Vorrichtung zum Anordnen einer Vorrichtung in einer Körperröhre, wie in einer Vene, in einer Arterie, im Harnleiter, im Gallengang oder dergleichen, vorgeschlagen, die im Hinblick auf ein unproblematisches, zuverlässiges und dauerhaftes Legen der Vorrichtung dadurch gekennzeichnet ist, daß das Drahtteil, als Rund- oder als Flachdraht, in axialer Richtung, mäanderförmig ausgebildet ist und die einzelnen Mäander (20) nahezu ringförmig gebogen sind.

## Beschreibung

Die Erfindung betrifft eine Vorrichtung nach dem Oberbegriff des Anspruchs 1.

Ein Verschluß in einem Gefäß, wie in einer Arterie, wird zunächst in der Regel erweitert, indem Ablagerungsmaterial, wie Plaques, entfernt wird, beispielsweise durch Herausschneiden und Absaugen oder dergleichen. Dies kann aber nicht bis zum Gewebematerial geschehen, da hier eine Verletzungsgefahr besteht. Wenn noch kein völliger Verschluß, sondern lediglich eine Verengung, eine Stenose, gebildet ist oder ein gewisser Durchflußbereich bei vorherigem vollständigen Verschluß in der vorgenannten Weise geschaffen wurde, ist daher eine radiale Aufweitung der Stenose gewünscht. Eine solche wird nicht nur in Blutgefäßen, sondern auch in anderen Körpergängen oder -röhren, wie im Harnleiter, Gallengang oder dergleichen, vorgenommen.

Hierzu wurde bisher ein sogenannter Ballonkatheter mit einem doppelten Lumen verwendet, der eine momentane radiale Aufweitung bewirkt, wobei diese verbleiben sollte, wenn der Ballonkatheter wieder entfernt wurde. Es wurden auch schon permanente Aufweitungen einer solchen Stenose bekannt. So wurde vorgeschlagen, auf einem derartigen Ballonkatheter ein Aufweiteteil axial fest aufzusetzen, mit dem Katheter einzuführen und durch Aufweiten des Ballonkatheters plastisch derart zu verformen, daß die radialen Abmessungen des Aufweiteteils sich vergrößern, dieses in die Wand der Stenose eingedrückt wird und nach Ablassen der Luft aus dem Ballonkatheter dieser entfernt werden kann, während das Aufweiteteil in plastisch verformtem Zustand an seinem Ort liegenbleibt. Als solches Teil wurde beispielsweise eine Manschette in Form einer "schraubenförmigen" Hülse vorgesehen.

Ein wesentlicher Nachteil des letztgenannten Verfahrens besteht darin, daß die Manschette auf dem Außenumfang eines Katheters aufsitzend in die Stenose eingebracht werden kann. Zunächst ist die axiale Festlegung aufwendig und entweder nicht zuverlässig zu erreichen, so daß das Teil beim Vorschieben des Katheters zurückbleiben kann oder aber ein späteres Lösen des Teils vom Katheter ist schwierig. Darüber hinaus kann ein außen auf einem Katheter sitzendes Aufweiteteil auch zu Beschädigungen fuhren. Schließlich muß die Aufweitung durch den Ballonkatheter bei einem derartigen Teil zunächst zu einer Radialerstreckung erfolgen, die wesentlich über der letztendlich wünschenswerten und zu erreichenden Radialerstreckung liegt, da derartige Teile, wie herkömmliche biokompatible Materialien, soweit sie eine ausreichende Festigkeit haben, um eine Stenose offen zu halten, einen erheblichen elastischen Verformungsbereich aufweisen, bevor eine bleibende plastische Verformung auf dem gewünschten Radius eintritt. Dies gilt insbesondere für ein, wie erwähnt, "schraubenförmig" geformtes Teil.

Der Erfindung liegt daher die Aufgabe zugrunde, eine hochbiegsame Vorrichtung zur Anordnung in einer Körperröhre zu schaffen, die unter Vermeidung der vorgenannten Nachteile bei einfacher und preiswerter Herstellungsmöglichkeit eine einfache, unproblematische und zuverlässige Plazierung in kurzer Zeit bei sicherem Halt in der Körperröhre erlaubt.

Erfindungsgemäß wird die genannte Aufgabe durch eine gattungsgemäße Vorrichtung mit den kennzeichnenden Merkmalen des Anspruchs 1 gelöst.

Wenn die Mäander in axialer Richtung Ausbiegungen aufweisen, so sieht eine bevorzugte Weiterbildung vor, daß die Ausbiegungen eines Mäanderschenkels mit einem benachbarten Mäanderschenkel verbunden sind. Die Verbindung kann insbesondere durch Lösten erfolgen. Wenn nicht sämtliche Ausbiegungen mit dem benachbarten Mäanderschenkel in der typischen Weise fest verbunden sind, so kann gemäß einer weiteren bevorzugten Ausgestaltung vorgesehen sein, daß die Verbindungskräfte der Verbindungen größer sind als die Wiegekräfte der Mäanderbiegungen. Hierdurch kann die erfindungsgemäße Vorrichtung, die auch als Endoprothese oder Stent zu bezeichnen ist, in den Bereichen, in denen die Mäander in axialer Richtung nicht durch Verbindungen, wie Lötstellen, fest miteinander verbunden sind, gedehnt oder gestaucht und damit in ihrer Länge angepaßt werden, ohne daß die an anderen Stellen vorhandenen Verbindungen (Lötungen) reißen oder brechen.

Andererseits sollen die Verbindungsstellen als definierte Bruchstellen ausgebildet sein und zwar dann, wenn ein in der vorbeschriebenen Weise ausgebildete Vorrichtung später wieder entfernt werden soll, so daß beim Ziehen am einen Ende des die Vorrichtung bildenden Drahtes die Verbindungsstellen aufbrechen, der Draht sich streckt und daher in bequemer Weise die Vorrichtung entfernt werden kann. Das Entfernen einer Vorrichtung in der vorstehend beschriebenen Ausgestaltung oder bei einer wendelförmig geformten Vorrichtung wird erleichtert, wenn, an mindestens einem Ende des die Vorrichtung bildenden Drahtes eine Kugel ausgebildet ist. Es kann dann an der Kugel mit einer Hohlzange angegriffen werden und die Streckung der Vorrichtung erreicht werden.

Während die Außenkontur der erfindungsgemäßen Vorrichtungen in der Regel zylindermantelförmig ist, sehen in gewissen Einsatzfällen äußerst bevorzugte Ausgestaltungen konusförmige oder doppelkonusförmige Außenkonturen vor.

Eine weitere bevorzugte Ausgestaltung sieht vor, daß das Metall des insoweit beschriebenen Katheters in gewebekompatible Kunststoffe oder vorzugsweise Silikon eingebettet ist, so daß trotz der Zwischenräume zwischen Bereichen des Metallmaterials ein geschlossenem Mantelbereich geschaffen wird. Hierdurch wird bei malignen Tumoren verhindert, daß Zellen ins Innere der Endoprothese einwachsen.

Die erfindungsgemäße Vorrichtung kann in vielfältigen Bereichen und zu vielfältigen Zwecken eingesetzt werden, so in der Urethra, im Ureter, im Harnblasenhals, in Dedukti Bilipheri, in Blutgefäßen, wie Arterien und Venen, auch als Thrombenfilter, im Ösophagus und in der Trachea sowie im Darmbereich, insbesondere im Intestinum Rektum. Die Erfindung bietet steife und hochflexible, insbesondere mit ihrer Achse biegbare Vorrichtungen an, letzteres gilt insbesondere für Vorrichtungen mit mäanderförmig geführtem Draht.

Die Erfindung schlägt in bevorzugter Ausgestaltung ein Aufweiteteil aus einer Formgedächtnis-Legierung vor, das oberhalb einer gewissen Übergangstemperatur, die unterhalb der Körpertemperatur liegt, und damit also bei der Körpertemperatur, eine Form einnimmt, die der gewünschten Aufweitung der Körperröhre entspricht. Eine bevorzugte Ausgestaltung sieht dabei vor, daß die Formgedächtnis-Legierung eine Nickel-Titan-Legierung ist. Weiterhin können aber auch Cu-Zn-Al- oder Cu-Al-Ni-Legierungen verwendet werden. Derartige Legierungen sind unter den Warenzeichen Nitinol, Biometall (Firma Toki, Japan) oder Memotal bekannt.

Die die Formänderung bewirkende martensitische Reaktion bzw. Umwandlung erfolgt durch Temperaturänderung. Während die Übergangstemperatur in weiten Bereichen liegen kann, soweit sie grundsätzlich unter der Körpertemperatur liegt, sieht eine bevorzugte Ausgestaltung vor, daß die Übergangstemperatur bei 20 °C liegt. Diese Temperatur ist hinreichend unterhalb der Temperatur, um eine zuverlässige Aufweitung des Teils aus Formgedächtnis-Legierung in seinen Hochtemperaturzustand zu bewirken. Gleichzeitig liegt die Temperatur so hoch, daß ein vom Operateur bei Umgebungstemperatur genommenes Teil, das damit in seinem Niedertemperaturzustand mit geringen Radialabmessungen ist, sich erst nach einer gewissen Zeit radial aufweitet, die ausreichend ist, um das Teil in den Körper und bis zu der Stenose einzuführen.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnungen im einzelnen erläutert sind. Dabei zeigt:
- Figur 1: eine schematische Darstellung einer Vorrichtung mit mäanderförmig geführtem Draht, wobei die einzelnen Mäander teilringförmig gebogen sind, mit zylindrischer Außenkontur; und
- Figur 2: eine Vorrichtung ähnlich der der Figur 1 mit doppelkonischer Außenkontur ebenfalls in schematischer Darstellung.

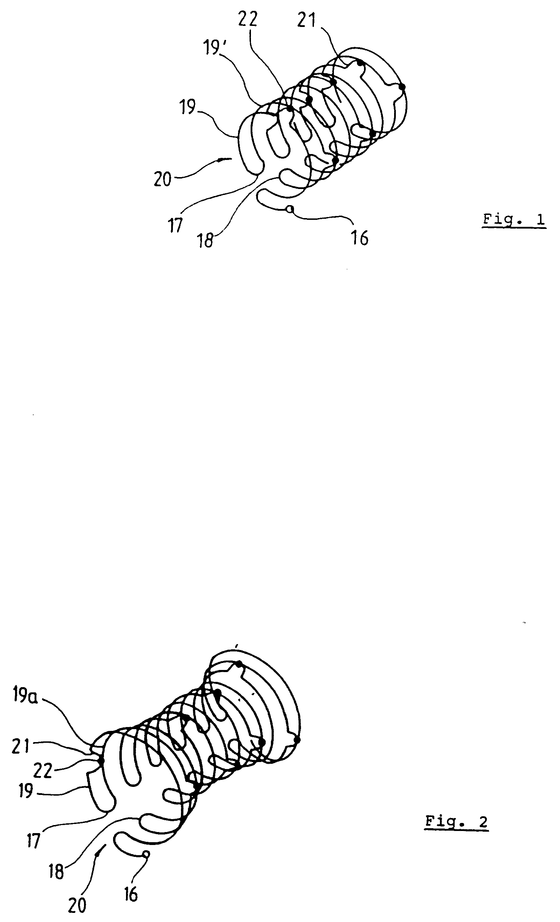

In der Figur 1 ist in einer ersten Ausgestaltung eine erfindungsgemäße Vorrichtung zum Anordnen in Körperröhren, wie in einer Vene, in einer Arterie, im Harnleiter, im Gallengang oder dergleichen, dargestellt.

Die Vorrichtungen der Figuren 1 und 2 bestehen aus mäanderförmig in Längsrichtung gewundenem Draht (die Mäander selbst erstrecken sich also quer zur Längsrichtung), wobei die Mäander ringförmig gebogen sind, so daß die einander abgewandten Stege benachbarter Mäander aufeinander Zugebogen sind. Eine derartige Vorrichtung weist den wesentlichen Vorteil auf, daß sie in ihrer Länge verändert werden kann, ohne daß hierdurch der Querschnitt wesentlich beeinflußt wird. Die Vorrichtung kann gegenüber ihrer Normallängserstreckung gestaucht oder gestreckt werden, wobei sie in der so erfolgten Stellung durch die Reibkräfte zwischen ihr und den umgebenden Gefäßwandungen gehalten wird.

Die erfindungsgemäße Vorrichtung besteht aus Metall mit einem Formgedächtnis, insbesondere aus Nickel-Titan-Legierungen, aber auch aus Cu-Zn-Al- oder Cu-Al-Ni. Formgedächtnislegierungen sind unter den Bezeichnungen Nitinol, Bimetall oder Memotal bekannt. Die Herstellung des Stents geschieht derart, daß der Stent zunächst in seine expandierte Hochtemperaturform der Figur 1 gebracht wird und anschließend einer Wärmebehandlung unterzogen wird. Nach Abkühlung unter die Übergangstemperatur wird die Vorrichtung bleibend in ihre kontrahierte Niedertemperaturform gebracht. Wenn sie über die Übergangstemperatur, die im Bereich von 30 bis 35 °C, vorzugsweise bei 35 °C liegt, wieder erwärmt wird, so "erinnert" sie sich an ihre aufgeweitete Hochtemperaturform und nimmt diese wieder an.

In bevorzugter Ausgestaltung weisen Schenkel 19 der Mäander 20 Ausbiegungen 21 auf, die bis zum benachbarten Mäanderschenkel - beispielsweise 19' - reichen. Zumindestens ein Teil der Ausbiegung 21 kann mit dem jeweils benachbarten Mäanderschenkel 19' durch eine Lötstelle 22 verbunden sein. Je nach Einsatzzweck können sämtliche Ausbiegungen mit benachbarten Mäanderschenkeln verbunden sein - in diesem Falle ist die Länge der Vorrichtung begrenzt; es können lediglich ein Teil der Ausbiegungen verbunden sein, während an einigen Ausbiegungen keine Lötstellen vorhanden sind - hierdurch kann die Länge in begrenztem Umfange verändert werden; schließlich können Vorrichtungen derart ausgebildet sein, daß keinerlei Ausbiegungen mit benachbarten Mäanderschenkeln fest verbunden sind; die Ausbiegungen bedingen eine gewisse Nachgiebigkeit in radialer bzw. angularer Richtung der Vorrichtung.

Die Vorrichtungen der Figuren 1 und 2 weisen in der dargestellten, konkreten Ausführungsform an mindestens einem Drahtende ein Kügelchen 16 auf, um die Vorrichtung durch Strecken des sie bildenden Drahtes zu entfernen. Wenn eine eingelegte Vorrichtung (Stent) aus irgendwelchen Gründen wieder entfernt werden soll, so kann mittels einer Hohlzange an der Kugel 16 angegriffen und gezogen werden, wodurch die Vorrichtung aufgezogen und gestreckt wird und so aus dem Körperhohlraum, in dem sie plaziert war, entfernt werden kann.

Wenn Lötstellen 22 vorhanden sind, so ist deren Befestigungskraft wesentlich geringer als die Reißkraft des Fadens; sie sollte aber größer sein als die Biegekraft des Drahtes im Bereich der Stege 17, 18 der Mäanderwindungen.

Die Vorrichtung kann zylindrische Außenkontur aufweisen, wie dies in Figur 1 skizziert ist; sie kann aber auch konische oder doppelkonische Gestalt aufweisen, wie dies in der Figur 2 der Fall ist, wobei hier der größere Durchmesser an den Stirnseiten der Vorrichtung ausgebildet ist.

Die erfindungsgemäße Vorrichtung gemäß den Figuren 1 und 2 weist eine sehr hohe Flexibilität auf. Die Vorrichtung der Figuren 1 und 2 ist praktisch vollständig biegbar. Die erfindungsgemäßen Metall-Vorrichtungen können in kompatible Kunststoffe oder vorzugsweise Silikon eingebettet werden, welches die gleiche Kontur wie der Stent selbst annimmt. Hierdurch kann verhindert werden, daß im Bereich einer derart augestalteten Vorrichtung ein maligner Tumor durch den Durchschnittsstent hindurch wandern kann.

Der vorstehende, durch Mäanderführung erhaltene Stent läßt sich in vielfältiger Weise und zu vielfältigen Verwendungszwecken einsetzen. Besonders vorteilhaft läßt sich ein solcher Stent als Harnröhrenstent im Bereich der Prostata zur Aufweitung des dortigen Harnröhrenbereichs bei vergrößerter Prostata einsetzen. Er paßt sich dabei insbesondere gut aufgrund seiner Flexibilität der doppelhahnförmigen Ausbildung der männlichen Harnröhre an. Durch seine Längenveränderlichkeit kann er so in der Länge verändert werden, daß sein rückwärtiges oder äußeres Ende nicht in den Bereich des externen Sphinkter zu liegen kommt, so daß der Stent dessen Funktion nicht beeinträchtigt. Insbesondere bei einer solchen Anwendung ist die oben beschriebene Möglichkeit der Extraktion des Stents durch Strecken des ihn bildenden Drahtfadens und Herausziehen äußerst vorteilhaft.

## Patentansprüche

1. Vorrichtung zur Anordnung in einer Körperröhre, wie in einer Vene, in einer Arterie, im Harnleiter, im Gallengang oder dergleichen, mit einem Drahtteil am Metallmaterial, dadurch gekennzeichnet, daß das Drahtteil, als Rund- oder als Flachdraht, in axialer Richtung, mäanderförmig ausgebildet ist und die einzelnen Mäander (20) nahezu ringförmig gebogen sind.

2. Vorrichtung nach Anspruch 1, dadurch gekennzeichnet, daß die Mäander (20) in axialer Richtung Ausbiegungen (21) aufweisen.

3. Vorrichtung nach Anspruch 2, dadurch gekennzeichnet, daß die Ausbiegungen eines Mäanderschenkels (19) mit einem benachbarten Mäanderschenkel (19) verbunden sind (bei 22).

4. Vorrichtung nach Anspruch 3, dadurch gekennzeichnet, daß die Verbindungskräfte der Verbindungen (22) größer sind als die Biegekräfte der Mäanderbiegungen (17, 18).

5. Vorrichtung nach Anspruch 3 oder 4, dadurch gekennzeichnet, daß die Verbindungskräfte der Verbindungen (22) geringer als die Reißkraft des Drahtes sind.

6. Vorrichtung nach einem der Ansprüche 1 bis 5, dadurch gekennzeichnet, daß an mindestens einem Ende des die Vorrichtung bildenden Drahtes eine Kugel (16) ausgebildet ist.

7. Vorrichtung nach einem der Ansprüche 1 bis 6, gekennzeichnet durch zumindest konusförmige Außenkontur.

8. Vorrichtung nach einem der Ansprüche 1 bis 7, gekennzeichnet durch doppelkonusförmige Außenkontur.

9. Vorrichtung nach einem der Ansprüche 1 bis 6, gekennzeichnet durch zylinderförmige Außenkontur.

10. Vorrichtung nach einem der vorangehenden Ansprüche, dadurch gekennzeichnet, daß das Metallmaterial in einen geschlossenen Mantel aus gewebekompatiblen Grundstoffen, insbesondere Silikon, eingebettet ist.

11. Vorrichtung nach einem der Vorangehenden Ansprüche, dadurch gekennzeichnet, daß das Metallmaterial eine Formgedächtnis-Legierung derart ist, daß sich das Drahtteil bei einer Übergangstemperatur, die oberhalb der Umgebungstemperatur, aber unterhalb der Körpertemperatur liegt, radial unter Beibehaltung einer zylindrischen Außenkontur aufweitet, wobei der Durchmesser bei einer Temperatur unterhalb der Übergangstemperatur unter dem Durchmesser des Gefäßes liegt.

12. Vorrichtung nach Anspruch 11, dadurch gekennzeichnet, daß die Formgedächtnis-Legierung eine Nickel-Titan-Legierung ist.

13. Vorrichtung nach Anspruch 11 oder 12, dadurch gekennzeichnet, daß die Übergangstemperatur zwischen 30 und 35 °C liegt.

14. Vorrichtung nach Anspruch 13, dadurch gekennzeichnet, daß die Übergangstemperatur bei 35 °C liegt.
